# EUROPEAN PATENT APPLICATION

(11) **EP 2 343 056 A1**
(43) Date of publication of application: **13.07.2011**
(21) Application number: 09180930.1
(22) Date of filing: 30.12.2009
(51) Int. Cl.: A61K 9/16, A61K 31/513, A61K 9/14

(54) **Producing stable adefovir dipivoxil solid dispersions**

(71) Applicant: Abdi Ibrahim Ilac Sanayi Ve Ticaret Anonim Sirketi, 34555 Istanbul (TR)
(72) Inventor: Farshi, Farhad, 34555, Istanbul (TR); Koc, Fikret, 34555, Istanbul (TR); Durmus, Semih, 34555, Istanbul (TR); Soylemez, Serdar, 34555, Istanbul (TR)

(57) **Abstract**

The present invention relates to a novel solid dispersion of Adefovir Dipivoxil (AD) containing amorphous form of AD as well as methods of producing them and pharmaceutical compositions containing said solid dispersions.

## Description

### Technical Field

The present invention relates to a novel solid dispersion of Adefovir Dipivoxil (AD) containing amorphous form of AD as well as methods of producing them and pharmaceutical compositions containing said solid dispersions. Conversion of crystalline AD to amorphous form can be achieved by inserting said drug molecule into carriers.

### Background Art

The solubility behaviour is one of the most important features about drug molecules. In recent years due to the development of increasing number of poorly soluble drug candidates, there is a great interest in solid dispersion systems to increase the dissolution rates and bioavailability of poorly soluble drug molecules. Solid dispersion systems can be obtained by many different methods. These are, but not limited to, melting method, solvent method, melting solvent method,melt extrusion method, lyophilisation technique, and electrospinning. Preferably solvent method is used. Although there are few products on the market obtained by using these methods, there is a great potential of commercialization of said systems with the introduction of new manufacturing technologies.

The present invention relates to medicinal compositions of a low-solubility crystalline drug and a high surface area substrate, wherein the drug and substrate are combined to form amorphous solid dispersion systems. The present invention is also related to a discovery that AD can be kept in novel stable amorphous form in the solid dispersion systems.

The nucleotide analogue reverse transcriptase inhibitor Adefovir Dipivoxil (AD) works by blocking the related enzyme that plays a major role for the reproduction of hepatitis B virus (HBV) in human body. AD is usually administered orally in the form of tablets containing crystalline AD. Adefovir Dipivoxil (in this application Adefovir Dipivoxil is abbreviated as AD) is presented by the following structure of formula 1:

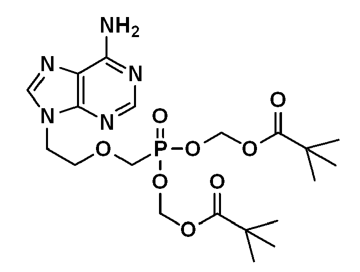

Its antitumor and antiviral activities were firstly reported with EP 0481214 B (INSTITUTE OF ORGANIC CHEMISTRY AND BIOCHEMISTRY OF THE ACADEMY OF SCIENCESOF THE CZECH REPUBLIC) . Further information about antiviral activities of AD can be obtained from STARRET, et al. Oral Bioavailability Determination, and in vitro Evaluation of Prodrugs of the Antiviral Agent 9-2-Phosphonomethoxy ethyl adenine . J. Med Chem. 1994, vol.37, p.1857-1864.

There are only a few crystalline and amorph form of AD present in the literature. It is well known that there are significant differences between the crystalline forms and the amorph form of the same compound. These differences may be related to melting point, solubility, density, flowability of said forms of pharmaceutical compounds. Based on the differences mentioned above, dissolution rate and stability of the related forms may be significantly different than each other.

WO 99/04774 A (GILEAD SCIENCES, INC.) discloses its crystalline form which is better for industrial applications. In the same patent, solvate forms and salts of AD were also reported along with two different crystalline forms.

First pharmaceutical formulations comprising anhydrous and dehydrate AD were disclosed in EP EP1140114 B (GILEAD SCIENCES, INC.) .

### Disclosure of Invention

Adefovir dipivoxil is readily available in its crystalline forms as it is known from the prior art.

The present invention is related to a production method for conversion of crystalline AD to amorphous form by inserting said drug molecule into carriers.

In a second aspect this invention is relevant to *ipso facto* non-convertible solid dispersions and formulations of amorphous AD to crystalline AD. The term *"ipso facto* non-convertible" means that without any treatment, amorphous state is maintained by itself in solid dispersion or in pharmaceutical composition or pharmaceutical combination which include solid dispersion.

In a third aspect, this invention provides amorphous AD with eligible flowability properties.

In a forth aspect dissolution properties of AD were significantly improved by its conversion to amorphous state by complexation with variety of carriers.

In a fifth aspect, when crystalline AD is treated with some carriers it is permanently converted to amorphous AD.

There are only few crystalline forms reported until now and there is a need for the development of new forms of AD, with better solubility and improved bioavailability properties in particular. These new forms can also be produced readily in industrial scale. Disclosed methods for the production of solid dispersions can be used to prepare AD solid dispersions in a wide variety of scales from milligrams to kilograms.

It is a well known fact how difficult is to develop absorptivity of poorly soluble drugs by conventional techniques used in the formulation studies. It has been previously reported that amorphous form of many drugs has improved dissolution properties over the crystalline forms. The requirement for more soluble AD form was met with the production of novel stable solid dispersions in amorphous state and a pharmaceutical composition comprising them.

AD is a drug molecule which is slightly soluble in water (19 mg/mL at pH 2.0). This solubility problem can be handled by using amorphous form of AD in pharmaceutical compositions. The process is described in the EP0481214 application and comprising dissolving 9-(2-Phosphonylmethoxy)ethyladenine (PMEA), N,N'-dicyclohexyl-4-morpholine carboxamidine and chloromethyl pivalate in anhydrous DMF and followed with the removal of the volatiles yielding amorphous AD.

The present invention provides solid state physical properties of AD which can be altered by the polymorphic form change obtained in solid form. Flowability is one of the major solid state property affects the ease of the drug's processability into a pharmaceutical product. During tabletting or capsule filling, there will be need for the use of glidants in case the powder does not flow fast enough. This efficient process for the production of amorphous ADsolid dispersions also eliminates the problems of prior art and readily applicable to the industrial scale. Thus according to the invention elegant flowability results are obtained by solid dispersion.

Dissolution properties of AD are also significantly improved by its conversion to amorphous state by complexation with variety of carriers. In addition to their improved solubility properties, they are also stable for extended period of times. These solids can be used for formulating wide variety of oral dosage forms such as tablets, capsules and suspensions etc. providing improved dissolution characteristics.

We have invented these novel solid dispersions of AD exhibiting good solubility, distribution and flowability. These properties make the said solid dispersion good candidates for large scale industrial production and also easy use of these in formulation studies.

According to this invention the list below is disclosed: A. amorphous solid dispersion characterized in that containing amorphous adefovir dipivoxil permanently remain its amorphous peculiarity and *ipso facto* non convertible to crystalline adefovir dipivoxil, B. amorphous solid dispersion comprising amorphous adefovir dipivoxil and an excipient or mixtures of excipients wherein adefovir dipivoxil is in crystalline form before conversion and used excipient or mixture of excipients have conversion properties from crystalline adefovir dipivoxil to amorphous adefovir dipivoxil and after treating crystalline adefovir dipivoxil with said excipient or mixture of excipients it is converted to amorphous adefovir dipivoxil , C. amorphous adefovir dipivoxil solid dispersion characterized in that containing amorphous adefovir dipivoxil is converted from crystalline adefovir dipivoxil and amorphous adefovir dipivoxil permanently remains its amorphous peculiarity and *ipso facto* non-convertible to crystalline adefovir dipivoxil, D. amorphous solid dispersion comprising amorphous adefovir dipivoxil and an excipient or mixtures of excipients wherein adefovir dipivoxil is in amorphous form and it remains amorphous peculiarity and *ipso facto* non-convertible to crystalline adefovir dipivoxil and used excipient or mixture of excipients have preventing properties from amorphous adefovir dipivoxil to crystalline adefovir dipivoxil, E. pharmaceutical composition comprising amorphous adefovir dipivoxil solid dispersion and preferably an excipient or mixture of excipients, F. pharmaceutical composition comprising amorphous adefovir dipivoxil solid dispersion and preferably an excipient or mixtures of excipients characterized in that containing amorphous adefovir dipivoxil permanently remain its amorphous peculiarity and *ipso facto* non convertible to crystalline adefovir dipivoxil,

Current therapies against viruses may require combination of two or more active pharmaceutical ingredients preferably therapeutic antivirals. There will be no interaction between other antiviral molecules and said solid dispersion systems comprising embedded AD into carriers. Therefore, the novel solid dispersion systems disclosed in the present invention may also be very effective in the use of developing fixed dose combination drug products. Thus this invention includes: **A.** pharmaceutical combination comprising amorphous adefovir dipivoxil solid dispersion and at least one active pharmaceutical ingredient and preferably an excipient or mixture of excipients, **B.** pharmaceutical combination comprising amorphous adefovir dipivoxil solid dispersion and at least one active pharmaceutical ingredient and preferably an excipient or mixture of excipients characterized in that containing amorphous adefovir dipivoxil permanently remain its amorphous peculiarity and *ipso facto* non convertible to crystalline adefovir dipivoxil.

According to this invention, in a one embodiment, solid dispersions of AD of the present invention were produced by a method below:
1. Dissolving a carrier or mixtures of carriers in an organic solvent or mixtures of organic solvents and
2. Dissolving crystalline AD in the organic solvent or mixtures of organic solvents or
3. Dissolving crystalline AD and excipient or mixtures of excipients in a solvent or mixtures of solvents and
4. Mixing of the solutions obtained from step 1 and step 2 if AD and carrier are not dissolved in same solvent and
5. Stirring at room temperature;
6. Removal of the volatiles to afford the powder amorphous AD solid dispersion.

Removal of volatiles in step 6 is performed at a temperature of about 25°C to about 80°C.

The process presented in this invention also includes the dissolution of AD and the related carrier in a common solvent mixture which dissolve both AD and carrier.

Organic solvent/solvent mixtures may be selected from the group including alcohols, alkyl halides, ethers, ketones and the like. Ethanol was mostly preferred due to its low toxicity and the ease of removal. It is also clear that other co-solvents for both AD and the related polymer may be also be used.

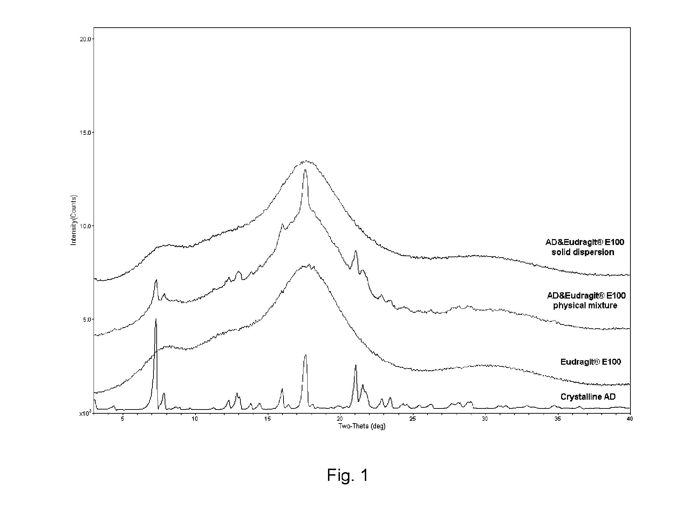

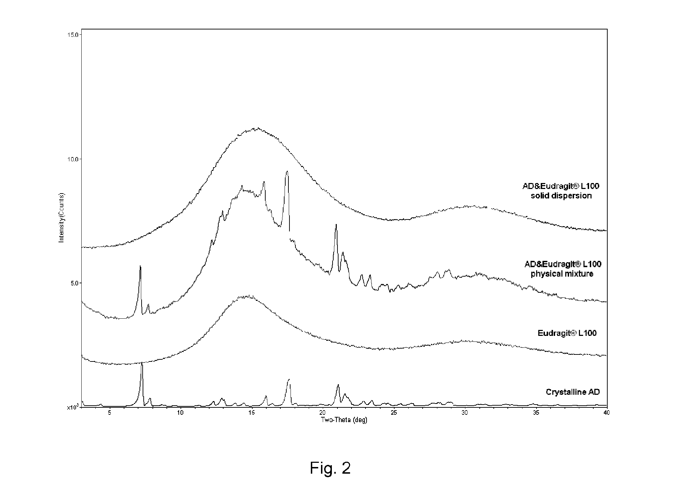

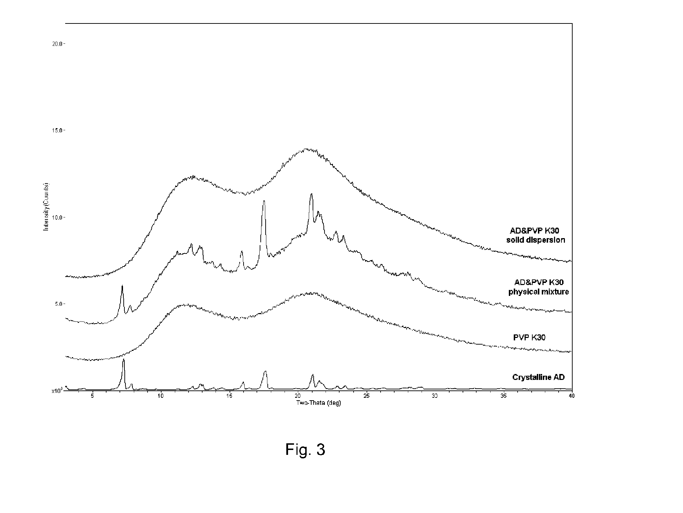

Amorphous AD containing solid dispersion systems prepared according to the process of the present invention are characterized by their powder x-ray diffraction pattern as shown in the related figures (Figure 1-3). There are no crystalline characteristic peaks observed in the diffraction patterns (Figure 1-3). These patterns only show the presence of amorphous AD in the related polymers. The x-ray diffraction patterns also showed that crystalline AD was dispersed homogeneously. Additionally there is no conversion from amorphous AD to crystalline AD. In solid dispersion or in pharmaceutical solid dispersions where solid dispersion is used in pharmaceutical formulations amorphous AD is permanently remained.

The excipients, in using of conversion crystalline AD to amorphous AD, are, but not limited to, Eudragit Ⓡ L100, Eudragit Ⓡ E100 and PVP K30, mixtures thereof and the like. Any suitable carrier or carriers can be used in conversion which provides conversion from crystalline AD to amorphous AD. In the solid dispersion weight ratio of AD to said carrier is about 1:99 to 99:1. Said carriers are preferably presented in the related solid dispersions the weight ratio of AD to said carrier is about from 1:1 to 1:10.

Said amorphous solid dispersions can also be obtained by using melting methods using low melting point excipients together with AD. Such excipients can be selected from the group of citric acid, tartaric acid, mixtures thereof and the like.

According to this invention, amorphous AD solid dispersion can be used in formulations of immediate release, quick release, sustained release, modified release, prolonged release and the like.

### Example 1

EudragitⓇ E100 was dissolved in acetone in a 100mL round bottom flask. While stirring, a solution of AD in acetone was added to the solution of Eudragit Ⓡ E100 and allowed to mix for 30 minutes at room temperature. The volatiles were removed in vacuo yielding white foamy material. The foam was subjected to high vacuum to remove the solvent residue.

### Example 2

AD Eudragit Ⓡ L100 solid dispersion was also prepared in the same manner as described in Example 1 by using following solutions: AD in Ethanol and Eudragit Ⓡ L100 in Ethanol.

### Example 3

AD PVP K30 solid dispersion was also prepared in the same manner as described in Example 1 by using following solutions: AD in dichloromethane and PVP K30 in dichloromethane.

### Characterization of Solid AD Dispersions

X-Ray powder diffraction spectroscopy was used for the characterization of each of the novel solid dispersions of adefovir dipivoxil. Based on the data obtained using this method, it is clear that all the novel solid dispersions contain amorphous adefovir dipivoxil.

### Instrumentation

The powder X-Ray diffraction patterns (PXRD) were collected on a Rigaku Ultima IV diffractometer with Cu Kα radiation generated at 40kV and 30mA. Data were collected from 3° to 40° (2*θ*) at a step size of 0.02° and a scanning speed of 0.3°/min.

## Claims

1. A solid dispersion containing amorphous adefovir dipivoxil.

2. An amorphous solid dispersion **characterized in that** containing amorphous adefovir dipivoxil permanently remain its amorphous peculiarity and *ipso facto* non convertible to crystalline adefovir dipivoxil.

3. An amorphous solid dispersion comprising amorphous adefovir dipivoxil and an excipient or mixtures of excipients wherein adefovir dipivoxil is in crystalline form before conversion and used excipient or mixture of excipients have conversion properties from crystalline adefovir dipivoxil to amorphous adefovir dipivoxil and after treating crystalline adefovir dipivoxil with said excipient or mixture of excipients it is converted to amorphous adefovir dipivoxil .

4. An amorphous adefovir dipivoxil solid dispersion **characterized in that** containing amorphous adefovir dipivoxil is converted from crystalline adefovir dipivoxil and amorphous adefovir dipivoxil permanently remains its amorphous peculiarity and *ipso facto* non-convertible to crystalline adefovir dipivoxil.

5. An amorphous solid dispersion comprising amorphous adefovir dipivoxil and an excipient or mixtures of excipients wherein adefovir dipivoxil is in amorphous form and it remains amorphous peculiarity and *ipso facto* non-convertible to crystalline adefovir dipivoxil and used excipient or mixture of excipients have preventing properties from amorphous adefovir dipivoxil to crystalline adefovir dipivoxil.

6. According to preceding claims excipient or mixtures of excipients are used as carrier.

7. According to claim 6, excipients used as carriers are selected from group consisting of Eudragit Ⓡ L100, Eudragit Ⓡ E100, PVP K30, mixtures thereof.

8. A process for the preparation of amorphous solid dispersion comprising: a. Dissolving crystalline AD in a solvent or mixture of solvents and b. Dissolving the excipient in a solvent or mixture of solvents or c. Dissolving crystalline AD and excipient or mixtures of excipients in a solvent or mixtures of solvents and d. Combining of solutions obtained from step (a) and step (b) if step (c) is not preferred and e. Removing the volatiles by evaporization yielding the solid dispersion product and

9. The process as claimed in claim 8, wherein the removal of volatiles in step e is performed at a temperature of 25°C to 80°C.

10. The process as claimed in claim 8, wherein the organic solvent/s used in step a, step b and step c is selected from the group consisting of methanol, ethanol, isopropanol, methylene chloride, mixtures thereof.

11. A pharmaceutical composition comprising amorphous adefovir dipivoxil solid dispersion and preferably an excipient or mixture of excipients.

12. A pharmaceutical composition comprising amorphous adefovir dipivoxil solid dispersion and preferably an excipient or mixtures of excipients **characterized in that** containing amorphous adefovir dipivoxil permanently remain its amorphous peculiarity and *ipso facto* non convertible to crystalline adefovir dipivoxil.

13. A pharmaceutical combination comprising amorphous adefovir dipivoxil solid dispersion and at least one active pharmaceutical ingredient and preferably an excipient or mixture of excipients.

14. A pharmaceutical combination comprising amorphous adefovir dipivoxil solid dispersion and at least one active pharmaceutical ingredient and preferably an excipient or mixture of excipients **characterized in that** containing amorphous adefovir dipivoxil permanently remain its amorphous peculiarity and *ipso facto* non convertible to crystalline adefovir dipivoxil.

15. The solid dispersion of any one of preceding claims wherein the weight ratio of AD to said carrier is 1:99 to 99:1.

16. According to claim 16, in the related solid dispersions the weight ratio of AD to said carrier is from 1:1 to 1:10.

17. The methods to obtain solid dispersion, as claimed in claim preceding claims, are selected from group consisting of melting method, solvent method, melting solvent method,melt extrusion method, lyophilisation technique, and electrospinning.

18. According to claim 16 the method to obtain solid dispersion is solvent method.
